# EUROPEAN PATENT SPECIFICATION

(11) **EP 0 833 925 B1**
(45) Date of publication and mention of the grant of the patent: **29.03.2006**
(21) Application number: 96920953.5
(22) Date of filing: 20.06.1996
(51) Int. Cl.: C12N 15/50, C07K 14/165, A61K 39/215, G01N 33/569

(54) **DIAGNOSTIC TEST FOR EQUINE ARTERITIS VIRUS MEDIATED DISEASE**
DIAGNOSTISCHER TEST FÜR VON PFERDEARTHRITISVIRUS VERURSACHTE KRANKHEITEN
TEST DIAGNOSTIQUE DE LA MALADIE INDUITE PAR LE VIRUS DE L'ARTERITE EQUINE

(30) Priority: 20.06.1995 GB 9512464
(43) Date of publication of application: 08.04.1998
(73) Proprietor: ANIMAL HEALTH TRUST, Newmarket, Suffolk CB8 7UU (GB)
(72) Inventor: CHIRNSIDE, Ewan, Douglas, Newmarket, Suffolk CB8 7PN (GB)
(74) Representative: Davies, Jonathan Mark
(86) International application number: PCT/GB1996/001505
(87) International publication number: WO 1997/000963

(56) References cited:
- WO-A-95/19438
- US-A- 3 590 127
- US-A- 5 202 430
- J GEN VIROL, JUN 1994, 75 ( PT 6) P1491-7, ENGLAND, XP002017231 CHIRNSIDE ED ET AL: "Comparison of M and N gene sequences distinguishes variation amongst equine arteritis virus isolates."
- J VIROL, JUN 1991, 65 (6) P2910-20, UNITED STATES, XP000607121 DEN BOON JA ET AL: "Equine arteritis virus is not a togavirus but belongs to the coronaviruslike superfamily."
- NUCLEIC ACIDS RES, JUN 11 1990, 18 (11) P3241-7, ENGLAND, XP002017233 DE VRIES AA ET AL: "All subgenomic mRNAs of equine arteritis virus contain a common leader sequence."
- J VIROL, NOV 1992, 66 (11) P6294-303, UNITED STATES, XP000607054 DE VRIES AA ET AL: "Structural proteins of equine arteritis virus."
- REV SCI TECH, SEP 1994, 13 (3) P845-54, FRANCE, XP000607117 ZIENTARA S: "[Equine infectious arteritis: molecular biology, epidemiology and preventative measures]"
- BRITISH VETERINARY JOURNAL, 1992, 148, 181-197, XP000607273 CHIRNSIDE ED: "EQUINE ARTERITIS VIRUS - AN OVERVIEW"
- VIRUS RESEARCH, 1995, 39, 277-288, XP000607654 CHIRNSIDE ED ET AL: "EXPRESSION CLONING AND ANTIGENIC ANALYSIS OF THE NUCLEOCAPSID PROTEIN OF EQUINE ARTERITIS VIRUS"
- J GEN VIROL, AUG 1995, 76 ( PT 8) P1989-98, ENGLAND, XP002017238 CHIRNSIDE ED ET AL: "Equine arteritis virus-neutralizing antibody in the horse is induced by a determinant on the large envelope glycoprotein GL."

## Description

The present invention relates to recombinant DNA and polypeptides encoded thereby, having use in provision of antibodies, vaccines, diagnostic test kits and methods of diagnosis and treatment or prophylaxis for equine arteritis virus (EAV) and equine arteritis virus mediated disease.

Equine viral arteritis, a disease for which equids are the only reported hosts, has been known for some 40 years and manifests itself with widely varying clinical signs. In its most severe form EAV infection causes abortion and foal death which makes it a potentially significant commercial threat to, inter alia, the horse breeding industry. Early veterinary articles refer to it as epizootic cellulitis, pinkeye or equine influenza. Disease outbreaks are identified infrequently and field isolates of the single stranded RNA virus itself are rare.

EAV is transmitted by the respiratory and venereal routes, with a 30-60% carrier state existing in seropositive stallions which persistently shed virus in their semen. Thus the venereal route is a particular cause for concern, as these shedding stallions may infect broodmares at mating. In the light of the potential economic importance of the virus and its stud carrier mediated infection capability there exists a requirement for prophylactic treatment and reliable diagnosis of equine viral arteritis, and rapid identification of equids previously exposed to the infectious agent.

Laboratory tests based on ELISA using whole virus as antigen, virus neutralisation (VN) and complement fixation (CF) formats have been developed (see Chirnside (1992) Br Vet J 148, pp 181). The known ELISA is relatively insensitive when applied to tissues, eg sera, from horses previously vaccinated for other diseases such as influenza and herpesvirus, while the VN and CF formats have limited temporal sensitivity; the VN test is unable to distinguish between vaccination and natural infection.

Vaccination procedures have concentrated on safety and efficacy of whole inactivated virus and attenuated live virus vaccines. The live vaccine can induce short term shedding of virus from the nasopharynx and does not prevent this causing infection of commonly housed animals which have not been so treated. It is not yet known if formalinised vaccines or other inactivated vaccine preparations provide reliable protection.

Attempts to provide improvements to both diagnostic tests and vaccines have included studies into panels of antibodies raised against various EAV proteins. A 29K envelope protein (G_{L}) has been identified as antigenic (Balasuriya *et al*, (1993) J Gen Virol 74, 2525-2529: Deregt et al, (1994) J Gen Virol 75, 2439-2444) and peptides derived from this protein induce a virus neutralising response in horses (Chimside et al, (1995) J Gen Virol in press). A previous invention (UK Patent Application No GB 9400656.4) provided isolated peptides of G_{L} that produce a potent neutralising immune response against EAV when administered to animals, particularly horses, and these peptides provided sensitive detection of EAV antibodies when used as binding agents in biding assays formats. Further provided was DNA encoding for these peptides.

In the first aspect of the present invention there is provided a method for testing for the presence of antibodies to equine arteritis virus comprising use of a peptide or peptide conjugate of the viral nucleocapsid (N) protein as a specific binding agent. Such test is preferably of ELISA format but may use the peptide or conjugate as immobilised binding agent or labelled secondary binding agent in a so-called sandwich assay. Preferred peptides or peptide conjugates of the invention comprise epitopes present in the amino acid sequence corresponding to amino acids 1 to 110 (SEQ ID No.2) of EAV N, more preferably amino acids 1 to 69 of EAV N (SEQ ID No.3) and, additionally, amino acids 1 to 28 (SEQ ID No.4), or a sequence having at least 90% homology to these sequences. These peptides are antigenic and may be used to provide isolated antibodies produced by an immunological response when a suitable host is exposed to the antigen.

In a binding assay where the peptide or peptide conjugate is immobilised this method may conveniently be carried out by use of commercially available assay plates onto which the peptide or conjugate is coated by suitable incubation in the known manner. For the purpose of assay, a sample to be screened for EAV antibodies, eg a serum sample, is typically incubated in contact with the plate, eg in the wells, whereafter any EAV antibody present is identified by exposure to eg an anti-horse IgA, IgM, IgG or other immunoglobulin conjugated to a reporter group. Such reporter group may be in the form of radiolabel, chemical label or a biological label. A typical biological label is an enzyme or cofactor, eg biotin, and is detected by exposure to all the reactants necessary for a reporter reaction to occur dependent upon the presence of the reporter group. In the case of biotin the well may be exposed to streptavidin-peroxidase and the substrate o-phenylenediamine dihydrochloride and the absorbance of the plate determined at 490nm.

In a further example, an immobilised anti-horse IgA, IgM, IgG or other immunoglobulin antibody raised in another animal, may be used to bind a specific class of horse antibody; the immobilised horse antibody provided may then be exposed to a solution containing labelled peptide or conjugate of the invention whereby the presence of anti-EAV antibody is indicated by assay of the amount of label present. Other assay formats such as competitive assays using either bound or unbound peptide or conjugate will occur to those skilled in the art; these will include simple observation of agglutination between peptide or conjugate and the antibody in a simple dilution test.

In a further aspect of the present invention there are provided test kits for use in carrying out the assay of the invention characterised in that they comprise a peptide, peptide-conjugate or antibodies of the invention, together with optional agents and items necessary for performing such assays. Such agents and items may include other binding agents or colour forming agents such as labelled antibodies eg biotinylated anti-horse IgG, horseradish peroxidase, streptavidin peroxidase conjugate and o-phenylenediamine dihydrochloride. It will be realised that the term peptide and peptide conjugate as used herein will encompass oligopeptides, polypeptides and proteins as long as they fulfill the criteria of the invention with regard to the immunological activity and content of epicopic sequences. The term conjugate designates conjugation to any physiologically acceptable entity.

The peptides, peptide conjugates and binding assays of the present invention will now be described, by way of example only, by reference to the following sequence listing, figures and examples.

### SEQUENCE LISTING

SEQ ID No.1: is the DNA sequence equivalent to the entire EAV genome minus the first 18 bases and the polyA tail.

SEQ ID No.2: is the amino acid sequence of the virus nucleocapsid protein (N) encoded by open reading frame (ORF) 7, and that is fused in-frame to GST in FP70 to express rN1-110.

SEQ ID No.3: is the amino acid sequence corresponding to amino acids 1 to 69 of the EAV N protein, and that is fused in-frame to GST in FP71 to express rN1-69.

SEQ ID No 4.: is the amino acid sequence corresponding to amino acids 1 to 28 of the EAV N protein, and that is fused in-frame to GST in FP7Fsp1 to express rN1-28.

### FIGURES

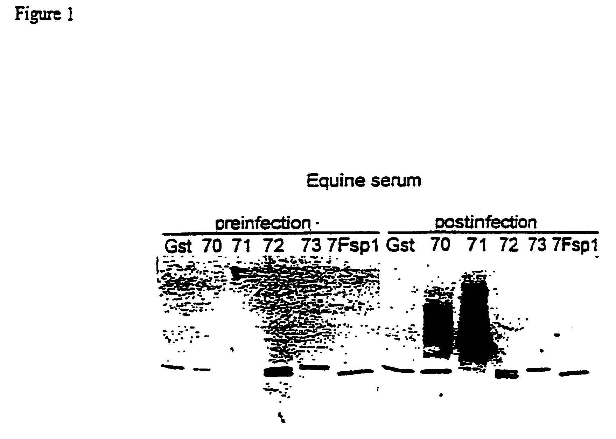

Figure 1 shows an immunoblot of purified fusion proteins (Fps 70, 71, 72, 73 and 7Fspl) and glutathione-S-transferase (Gst) with serum from an individual horse pre-and post-EAV infection. For plasmid and fusion protein derivations see Table 1.

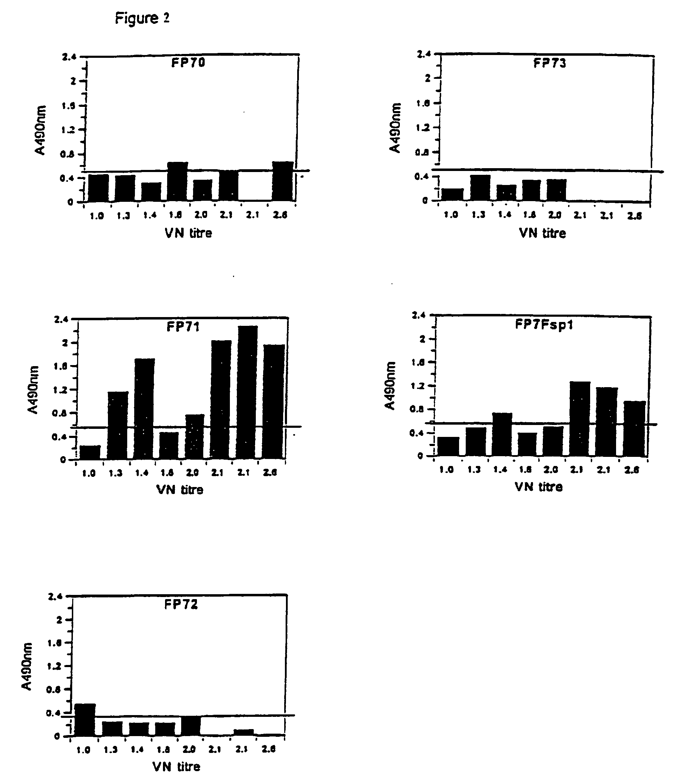

Figure 2 shows equine sera ELISA absorbance values to recombinant EAV N proteins. ELISA plates were coated with 0.5 *µ*g per well of purified fusion protein (FP) or glutathione-S-transferase (GST). Sera were tested in two replicate wells to each antigen and the absorbance of each well read at 490nm. The GST absorbance was subtracted from the FP absorbance to derive an EAV-specific value. Each bar represents the mean value from two replicates of each serum. Cut-off points determining ELISA seropositivity for each antigen, calculated from the absorbance values of 8 VN- control sera are shown as a horizontal line on each graph: FP70=0.592; FP71=0.483; FP72=0.294; FP73=0.407; FP7Fspl=0.582. The virus neutralising titre (VN titre) of the 8 sera tested are shown on the x-axis as log₁₀ VN litres.

### EXAMPLE 1: Production of peptides and conjugates of the invention and DNA and vectors encoding therefore.

cDNA encompassing EAV open reading frame (ORF) 7 (den Boon *et al* [1991], J Virol, 65, 2910-2920; de Vries et al. [1992] , J Virol 66, 6294-6303) corresponding to the EAV N protein was cloned into the bacterial vectors pGEX-3X and pGEX-2T (Table 1) and constructs screened for fusion protein expression using PAGE with cloning confirmed by RE digestion analysis and sequencing over the plasmid/insert junctions. Plasmids are referred to as FPx, the expressed recombinant fusion proteins as rNy-z (where y and z refer to amino acid residue numbers in EAV N). Affinity purified glutathione-S-transferase (GST) fusion proteins were screened for reactivity in immunoblots with a panel of pre- and post-EAV-infection equine sera. Although horse sera exhibit some background absorbence to GST in immunoblots, post-infection sera bound strongly and specifically to fusion proteins containing amino acids N1-110 and N1-69, and failed to bind fusion proteins containing N1-28, N70-89 and N90-110 specifically (Figure 1) .

### EXAMPLE 2: ELISA using EAV nucleocapsid (N) fusion proteins

Dynatech Immulon 3 microtitre plates were coated with rN1-69 or rN1-28 antigen by exposure to 100µl of 5*µ*g/ml antigen in 0.05M carbonate buffer at pH9.6 (Sigma cat No C3041) at 4°C overnight.

Plates were washed three times with phosphate buffered saline (PBS) containing 0.05% Tween^{D} 20 (PBST) and then blocked with 100µl PBST containing 5% normal goat serum (Seralab) (PBSTG) for 1 hour at 37°C. Plates were washed again three times with PBST to render them ready for use.

Test sera were diluted 1:100 in PBSTG and 100µl of this solution added to wells prepared as above and incubated for 90 minutes at 37°C. Plates were washed again three times with PBST and solution prepared by diluting goat anti-horse IgG biotin conjugate (KPL catalogue No 162102) 1:1000 in PBSTG and adding 100µl to each well before being incubated for 90 minutes at 37°C. Plates were washed three times with PBST and a solution prepared by diluting streptavidin-peroxidase conjugate (KPL catalogue No 143000) 1:1000 in PBSTG and adding 100*µ*l to each well before incubating at room temperature for 30 minutes. Plates were washed three times with PBST and 100*µ*l 0-phenylenediamine dihydrochloride (Sigma cat. No P8287) (0.5 mg/ml in 0.05 phosphate citrate buffer, pH5.0, Sigma cat. No. P4922)) added to each well and incubated for 10 minutes at room temperature. 50*µ*l 4M H₂SO₄ was added to stop the reaction and absorbence read at 490nm. Since horse sera at a 1:100 dilution can bind native GST it is necessary to subtract absorbence readings obtained for sera against GST from the GST-fusion protein absorbence. Each serum is tested in duplicate wells against each antigen.

Figure 2 shows the results of 8 VN equine sera in ELISA to different recombinant EAV N proteins. Cut-off points determining ELISA seropositivity for each antigen, calculated from the value of 8VN negative equine sera, are shown as a horizontal line on each graph (rN1-110 = 0.592; rN1-69 = 0.483; rN70-89 = 0.294; rN90-110 = 0.407; rN1-28 = 0.582). From these results rN1-69 and rN1-28 were identified as suitable antigens for the detection of EAV-specific antibodies in ELISA.

### EXAMPLE 3: ELISA using rN1-69 and rN1-28 binding agents

Panels containing seronegative and virus neutralising sera were tested in ELISA to purified rN1-69 and rN1-28 (Table 2). In ELISAs a recombinant fusion protein containing residues 1-69 or 1-28 discriminated between pre- and post-infection equine sera. In additional ELISA tests screening pre- and post-EAV vaccination samples and including isolate specific sera, the rN1-69 and rN1-28 antigens were able to discriminate between samples pre-and post vaccination with Artervac (commercial inactivated virus vaccine), even in the absence of vaccination induced neutralising antibody, and detect isolate-specific VN sera as seropositive in ELISA. The mean absorbence rising following vaccination were 1.240±0.690 and 0.495±0.352 for rN1-69 and rN1-28 respectively.

**Table 1. Nucleocapsid gene constructs and fusion proteins**

| Plasmid (FP) | Amino acid residue (N) | Fusion protein (rN) | Fusion protein size (kDa) | EAV cDNA clone | Restruction digest | pGEX vector restriction digest |
|---|---|---|---|---|---|---|
| 70 | -3¹-110 | 1-110 | 42 | 106² | HindIII (12305) - HindIII^{v}(>12700) | 3X x HindIII |
| 71 | -3¹-69 | 1-69 | 36 | FP70 | HindIII (12305) - RsaI (12523) | 3X x BamhI EcoRI^{E} |
| 72 | 70-89 | 70-89 | 30 | FP70 | RsaI (12524) - RsaI (12583) | 2T x SmaI |
| 73 | 90-10 | 90-110 | 30 | FP70 | RsaI (12584) - EcoRI" (.12700) | 2T x SmaI |
| 7Fsp1 | -3¹-28 | 1-28 | 31 | 106² | HindIII' (12305) - FspI (12399) | 3X x SmaI |

| | | | | | | |
|---|---|---|---|---|---|---|
| ^{r}3' recessed end filled in with the Klenow fragment of DNA polymerase | | | | | | |
| ^{v} Vector derived | | | | | | |
| ¹ The negative number corresponds to additional amino acids cloned into pGEX which are not encoded by ORF 7 | | | | | | |
| ² see de Vries *et al* 1990, Nuclei Acids Research 18. 3241-3247. | | | | | | |

**Table 2. Comparison of virus neutralising antibody titres and ELISA absorbence values**

| Equine sera | Log₁₀ VN antibody | VN test result | rN1-69 ELISA A,o | rN1-69 ELISA result" | rN1-28 ELISA λ₄₉₀ | rN1-28 ELISA result" |
|---|---|---|---|---|---|---|
| Negative controls | | | | | | |
| 32277 | 0 | - | 0.126 | - | 0.170 | - |
| 32278 | 0 | - | 0.156 | - | 0.310 | + |
| 32279 | 0 | - | 0.096 | - | 0.160 | - |
| 32280 | 0 | - | 0.095 | - | 0.101 | - |
| 32281 | 0 | - | 0.115 | - | 0.155 | - |
| 32282 | 0 | - | 0.115 | - | 0.134 | - |
| 32283 | 0 | - | 0.161 | - | 0.204 | - |
| 32284 | 0 | - | 0.152 | - | 0.222 | - |

### Post infection

| | | | | | | |
|---|---|---|---|---|---|---|
| 32252 | 3.6 | - | 3.746 | - | 2.250 | + |
| 32255 | 2.475 | - | 2.504 | - | 0.679 | + |
| 32257 | 2.625 | - | 3.660 | - | 1.856 | + |
| 32256 | 2.700 | + | 3.520 | - | 1.182 | + |
| 32259 | 2.550 | - | 2.536 | - | 0.650 | + |
| 32260 | 2.850 | - | 1.238 | - | 0.314 | - |
| 32261 | 1.875 | + | 2.753 | + | 2.024 | + |
| 32262 | 2.475 | + | 3.00 | + | 0.920 | + |

### Paired vaccination samples

| | | | | | | | |
|---|---|---|---|---|---|---|---|
| 33745 pre | | 0 | - | 0.132 | - | 0.230 | - |
| | post | 0.3 | - | 0.664 | - | 0.340 | - |
| 33746 pre | | 0 | - | 0.353 | + | 0.344 | + |
| | post | 0.45 | - | 0.967 | + | 0.580 | + |
| 33747 pre | | 0 | - | 0.1.68 | - | 0.256 | - |
| | post | 0.525 | - | 2.427 | + | 1.212 | + |
| 33962 pre | | 0 | - | 0.157 | - | 0.170 | - |
| | post | 0 | - | 0.884 | - | 0.397 | + |
| 33963 pre | | 0 | - | C.117 | - | 0.145 | - |
| | post | 0.9 | - | 2.144 | + | 0.997 | + |
| 33964 pre | | 0 | - | 0.156 | - | 0.175 | - |
| | post | 0 | - | 1.572 | - | 0.851 | + |
| 33435 pre | | 0 | - | 0.348 | - | 0.286 | - |
| | post | 0.3 | - | 1.452 | * | 0.491 | + |
| 35097 post | | 1.5. | | 3.226 | + | 2.026 | - |
| 35098 post | | 1.5 | - | 2.441 | - | 1.908 | + |

### Isolate specific

| | | | | | | |
|---|---|---|---|---|---|---|
| **Bucyrus** | 3.1 | - | 3.249 | - | 1.130 | - |
| 84-KY-A1 | 2.5 | - | 0.888 | - | 0.288 | - |
| Wroclaw-2 | 2.2 | - | 0.424 | - | 0.276 | - |
| Arvac | 2.5 | - | 0.422 | - | 0.319 | - |
| Killed Bucyrus | 1.9 | - | 1.117 | - | 0.620 | - |

| | | | | | | |
|---|---|---|---|---|---|---|
| ^{a}Log₁₀ VN antibody titre 2 0.6 is deemed seropositive in the EAV VN neutralising test | | | | | | |
| ^{b}the cut off value to determine seropositive status was taken as (mean +2SD) of the 8 VN negative control sera (positive ≥ 0.177) | | | | | | |
| ^{c}the cut off value to determine seropositive status was taken as (mean + 2SD) of the 8 VN negative control sera (positive ≥ 0.308) | | | | | | |
| pre - pre-vaccinacion serum sample | | | | | | |
| post = post-vaccination serum sample | | | | | | |

### SEQUENCE LISTING

**(1)** GENERAL INFORMATION:
   (i) APPLICANT:
      (A) NAME: The Animal Health Trust
      (B) STREET: PO Box 5
      (C) CITY: Newmarket
      (D) STATE: Suffolk
      (E) COUNTRY: United Kingdom
      (F) POSTAL CODE (ZIP): CB8 7DW
   (ii) TITLE OF INVENTION: Diagnostic Test For Equine Arteritis Virus Mediated Disease
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentin Release #1.0, Version #1.30 (EPO)
## Claims

1. An antigenic peptide fragment of EAV N (SEQ ID NO: 2) which fragment consists of:
a) amino acid residues 1 to 69 of EAV N (SEQ ID NO: 3), or
b) amino acid residues 1 to 28 of EAV N (SEQ ID NO: 4), or
c) a sequence of amino acid residues having at least 90% homology to any one of sequences (a) or (b).

2. A DNA sequence encoding an amino acid sequence selected from the group which consists of:
a) amino acid residues 1 to 69 of EAV N (SEQ ID NO: 3)
b) amino acid residues 1 to 28 of EAV N (SEQ ID NO: 4)
c) a sequence of amino acid residues having at least 90% homology to any one of sequences (a) or(b),

3. A composition which comprises as an active ingredient an antigenic peptide fragment according to claim 1 conjugated to a physiologically acceptable entity.

4. A method of assay of a sample to be screened for the presence of antibodies to equine arteritis virus (EAV) which includes the step of using a peptide according to claim 1 or peptide conjugate according to claim 3 as a specific binding agent for EAV antibody in the sample, and which method excludes the step of obtaining the sample.

5. A method according to claim 4 wherein the method uses the peptide or conjugate in an ELISA, or the peptide or peptide conjugate is an immobilised binding agent or labelled secondary binding agent in a sandwich assay.

6. A test kit for use in a method according to claim 4 which kit includes a peptide as defined in claim 1 or a conjugate according to claim 3.

7. An isolated antibody produced by an immunological response to a peptide as defined in claim 1 or a peptide conjugate according to claim 3.

8. A test kit for use in a method for testing for the presence of antibodies to equine arteritis virus (EAV) which kit includes an antibody according to claim 7.

## Revendications

1. Fragment peptidique antigénique de EAV N (SEQ ID NO : 2) lequel fragment consiste en :
a) les résidus d'acides aminés 1 à 69 de EAV N (SEQ ID NO : 3), ou
b) les résidus d'acides aminés 1 à 28 de EAV N (SEQ ID NO : 4) ; ou
c) une séquence de résidus d'acides aminés ayant au moins 90% d'homologie avec l'une quelconque des séquences (a) ou (b).

2. Séquence d'ADN codant une séquence d'acides aminés sélectionnés parmi le groupe qui consiste en :
a) les résidus d'acides aminés 1 à 69 de EAV N (SEQ ID NO : 3)
b) les résidus d'acides aminés 1 à 28 de EAV N (SEQ ID NO : 4)
c) une séquence de résidus d'acides aminés ayant au moins 90% d'homologie avec l'une quelconque des séquences (a) ou (b).

3. Composition qui comprend en tant que principe actif, un fragment peptidique antigénique selon la revendication 1, conjugué à une entité physiologiquement acceptable.

4. Méthode d'essai d'un échantillon à analyser pour la présence d'anticorps contre le virus de l'artérite équine (EAV), qui comprend l'étape consistant à utiliser un peptide selon la revendication 1 ou un conjugué peptidique selon la revendication 3 en tant qu'agent de liaison spécifique pour l'anticorps EAY dans l'échantillon, et laquelle méthode exclut l'étape consistant à obtenir l'échantillon.

5. Méthode selon la revendication 4, où la méthode utilise le peptide ou le conjugué peptidique dans un ELISA, ou bien le peptide ou le conjugué peptidique est un agent de liaison immobilisé ou un agent de liaison secondaire marqué dans un essai en sandwich.

6. Kit de test à utiliser dans le cadre d'une méthode selon la revendication 4, lequel kit comprend un peptide tel que défini dans la revendication 1, ou un conjugué selon la revendication 3.

7. Anticorps isolé produit par une réponse immunologique à un peptide tel que défini dans la revendication 1 ou à un conjugué peptidique selon la revendication 3.

8. Kit de test à utiliser dans le cadre d'une méthode de test pour la présence d'anticorps contre le virus de l'artérite équine (EAV), lequel kit comprend un anticorps selon la revendication 7.

## Patentansprüche

1. Antigenpeptidfragment von EAV N (SEQ ID Nr. 2), wobei das Fragment aus Folgendem besteht:
a) den Aminosäureresten 1 bis 69 von EAV N (SEQ ID Nr. 3) oder
b) den Aminosäureresten 1 bis 28 von EAV N (SEQ ID Nr. 4) oder
c) einer Sequenz von Aminosäureresten mit wenigstens 90 % Homologie zu einer beliebigen der Sequenzen (a) oder (b) .

2. DNA-Sequenz, die eine Aminosäuresequenz kodiert, ausgewählt aus der Gruppe bestehend aus:
a) den Aminosäureresten 1 bis 69 von EAV N (SEQ ID Nr. 3),
b) den Aminosäureresten 1 bis 28 von EAV N (SEQ ID Nr. 4),
c) einer Sequenz von Aminosäureresten mit wenigstens 90 % Homologie zu einer beliebigen der Sequenzen (a) oder (b).

3. Zusammensetzung, die als Wirkstoff ein Antigenpeptidfragment nach Anspruch 1 umfasst, das mit einer physiologisch akzeptablen Entität konjugiert ist.

4. Verfahren zur Untersuchung einer Probe, die im Hinblick auf die Anwesenheit von Antikörpern gegen das Equine Arteritis-Virus (EAV) gescreent werden soll, umfassend den Schritt des Verwendens eines Peptids nach Anspruch 1 oder eines Peptidkonjugats nach Anspruch 3 als spezifisches Bindungsmittel für EAV-Antikörper in der Probe, wobei das Verfahren den Schritt des Gewinnens der Probe ausschließt.

5. Verfahren nach Anspruch 4, wobei das Verfahren das Peptid oder Konjugat in einem ELISA verwendet oder das Peptid oder Peptidkonjugat ein immobilisiertes Bindungsmittel oder ein markiertes Sekundärbindungsmittel in einem Sandwich-Test ist.

6. Testkit zur Verwendung in einem Verfahren nach Anspruch 4, wobei der Kit ein Peptid gemäß der Definition in Anspruch 1 oder ein Konjugat nach Anspruch 3 beinhaltet.

7. Isolierter Antikörper, der durch eine immunologische Reaktion auf ein Peptid gemäß Definition in Anspruch 1 oder ein Peptidkonjugat nach Anspruch 3 produziert wird.

8. Testkit zur Verwendung in einem Verfahren zum Testen im Hinblick auf die Anwesenheit von Antikörpern gegen Equines Arteritis-Virus (EAV), wobei der Kit einen Antikörper gemäß Anspruch 7 beinhaltet.


(2) INFORMATION FOR SEQ ID NO: 1:
   (i) SEQUENCE CHARACTERISTICS:
      (A) LENGTH: 12687 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 1:
   TGCCATATAC GGCTCACCAC CATATACACT GCAAGAATTA CTATTCTTGT GGGCCCCTCT 60
   CGGTAAATCC TAGAGGGCTT TCCTCTCGTT ATTGCGAGAT TCGTCGTTAG ATAACGGCAA 120
   GTTCCCTTTC TTACTATCCT ATTTTCATCT TGTGGCTTGA CGGGTCACTG CCATCGTCGT 180
   CGATCTCTAT CAACTACCCT TGCGACTATG GCAACCTTCT CCGCTACTGG ATTTGGAGGG 240
   AGTTTTGTTA GGGACTGGTC CCTGGACTTA CCCGACGCTT GTGAGCATGG CGCGGGATTG 300
   TGCTGCGAAG TGGACGGCTC CACCTTATGC GCCGAGTGTT TTCGCGGTTG CGAAGGAATG 360
   GAGCAATGTC CTGGCTTGTT CATGGGACTG TTAAAACTGG CTTCGCCAGT TCCAGTGGGA 420
   CATAAGTTCC TGATTGGTTG GTATCGAGCT GCCAAAGTCA CCGGGCGTTA CAATTTCCTT 480
   GAGCTGTTGC AACACCCTGC TTTCGCCCAG CTGCGTGTGG TTGATGCTAG GTTAGCCATT 540
   GAAGAGGCAA GTGTGTTTAT TTCCACTGAC CACGCGTCTG CTAAGCGTTT CCCTGGCGCT 600
   AGATTTGCGC TGACACCGGT GTATGCTAAC GCTTGGGTTG TGAGCCCGGC TGCTAACAGT 660
   TTGATAGTGA CCACTGACCA GGAACAAGAT GGGTTCTGCT GGTTAAAACT TTTGCCACCT 720
   GACCGCCGTG AGGCTGGTTT GCGGTTGTAT TACAACCATT ACCGCGAACA AAGGACCGGG 780
   TGGCTGTCTA AAACAGGACT TCGCTTATGG CTTGGAGACC TGGGTTTGGG CATCAATGCG 840
   AGCTCTGGAG GGCTGAAATT CCACATTATG AGGGGTTCGC CTCAGCGAGC TTGGCATATC 900
   ACAACACGCA GCTGCAAGCT GAAGAGCTAC TACGTTTGTG ACATCTCTGA AGCAGACTGG 960
   TCCTGTTTGC CTGCTGGCAA CTACGGCGGC TACAATCCAC CAGGGGACGG AGCTTGCGGT 1020
   TACAGGTGCT TGGCCTTCAT GAATGGCGCC ACTGTTGTGT CGGCTGGTTG CAGTTCTGAC 1080
   TTGTGGTGTG ATGATGAGTT GGCTTATCGA GTCTTTCAAT TGTCACCCAC GTTCACGGTT 1140
   ACCATCCCAG GTGGGCGAGT TTGTCCGAAT GCCAAGTACG CAATGATTTG TGACAAGCAG 1200
   CACTGGCGCG TCAAACGTGC AAAGGGCGTC GGCCTGTGTC TCGATGAAAG CTGTTTCAGG 1260
   GGCATCTGCA ATTGCCAACG CATGAGTGGA CCACCACCTG CACCCGTGTC AGCCGCCGTG 1320
   TTAGATCACA TACTGGAGGC GGCGACGTTT GGCAACGTTC GCGTGGTTAC ACCTGAAGGG 1380
   CAGCCACGCC CCGTACCAGC GCCGCGAGTT CGTCCCAGCG CCAACTCTTC TGGAGATGTC 1440
   AAAGATCCGG CGCCCGTTCC GCCAGTACCA AAACCAAGGA CCAAGCTTGC CACACCGAAC 1500
   CCAACTCAGG CGCCCATCCC AGCACCGCGC ACGCGACTTC AAGGGGCCTC AACACAGGAG 1560
   CCACTGGCGA GTGCAGGAGT TGCTTCTGAC TCGGCACCTA AATGGCGTGT GGCCAAAACT 1620
   GTGTACAGCT CCGCGGAGCG CTTTCGGACC GAACTGGTAC AACGTGCTCG GTCCGTTGGG 1680
   GACGTTCTTG TTCAAGCGCT ACCGCTCAAA ACCCCAGCAG TGCAGCGGTA TACCATGACT 1740
   CTGAAGATGA TGCGTTCACG CTTCAGTTGG CACTGCGACG TGTGGTACCC TTTGGCTGTA 1800
   ATCGCTTGTT TGCTCCCTAT ATGGCCATCT CTTGCTTTGC TCCTTAGCTT TGCCATTGGG 1860
   TTGATACCCA GTGTGGGCAA TAATGTTGTT CTGACAGCGC TTCTGGTTTC ATCAGCTAAT 1920
   TATGTTGCGT CAATGGACCA TCAATGTGAA GGTGCGGCTT GCTTAGCCTT GCTGGAAGAA 1980
   GAACACTATT ATAGAGCGGT CCGTTGGCGC CCGATTACAG GCGCGCTGTC GCTTGTGCTC 2040
   AATTTACTGG GGCAGGTAGG CTATGTAGCT CGTTCCACCT TTGATGCAGC TTATGTTCCT 2100
   TGCACTGTGT TCGATCTTTG CAGCTTTGCT ATTCTGTACC TCTGCCGCAA TCGTTGCTGG 2160
   AGATGCTTCG GACGCTGTGT GCGAGTTGGG CCTGCCACGC ATGTTTTGGG CTCCACCGGG 2220
   CAACGAGTTT CCAAACTGGC GCTCATTGAT TTGTGTGACC ACTTTTCAAA GCCCACCATC 2280
   GATGTTGTGG GCATGGCAAC TGGTTGGAGC GGATGTTACA CAGGAACCGC CGCAATGGAG 2340
   CGTCAGTGTG CCTCTACGGT GGACCCTCAC TCGTTCGACC AGAAGAAGGC AGGAGCGACT 2400
   GTTTACCTCA CCCCCCCTGT CAACAGCGGG TCAGCGCTGC AGTGCCTCAA TGTCATGTGG 2460
   AAGCGACCAA TTGGGTCCAC TGTCCTTGGG GAACAAACAG GAGCTGTTGT GACGGCGGTC 2520
   AAGAGTATCT CTTTCTCACC TCCCTGCTGC GTCTCTACCA CTTTGCCCAC CCGACCCGGT 2580
   GTGACCGTTG TCGACCATGC TCTTTACAAC CGGTTGACTG CTTCAGGGGT CGATCCCGCT 2640
   TTATTGCGTG TTGGGCAAGG TGATTTTCTA AAACTTAATC CGGGGTTCCG GCTGATAGGT 2700
   GGATGGATTT ATGGGATATG CTATTTTGTG TTGGTGGTTG TGTCAACTTT TACCTGCTTA 2760
   CCTATCAAAT GTGGCATTGG CACCCGCGAC CCTTTCTGCC GCAGAGTGTT TTCTGTACCC 2820
   GTCACCAAGA CCCAAGAGCA CTGCCATGCT GGAATGTGTG CTAGCGCTGA AGGCATCTCT 2880
   CTGGACTCTC TGGGGTTAAC TCAGTTACAA AGTTACTGGA TCGCAGCCGT CACTAGCGGA 2940
   TTAGTGATCT TGTTGGTCTG CCACCGCCTG GCCATCAGCG CCTTGGACTT GTTGACTCTA 3000
   GCTTCCCCTT TAGTGTTGCT TGTGTTCCCT TGGGCATCTG TGGGGCTTTT ACTTGCTTGC 3060
   AGTCTCGCTG GTGCTGCTGT GAAAATACAG TTGTTGGCGA CGCTTTTTGT GAATCTGTTC 3120
   TTTCCCCAAG CTACCCTTGT CACTATGGGA TACTGGGCGT GCGTGGCGGC TTTGGCCGTT 3180
   TACAGTTTGA TGGGCTTGCG AGTGAAAGTG AATGTGCCCA TGTGTGTGAC ACCTGCCCAT 3240
   TTTCTGCTGC TGGCGAGGTC AGCTGGACAG TCAAGAGAGC AGATGCTCCG GGTCAGGGCT 3300
   GCTGCCCCCA CCAATTCACT GCTTGGAGTG GCTCGTGATT GTTATGTCAC AGGCACAACT 3360
   CGGCTGTACA TACCCAAGGA AGGCGGGATG GTGTTTGAAG GGCTATTCAG GTCACCGAAG 3420
   GCGCGCGGCA ACGTCGGCTT CGTGGCTGGT AGCAGCTACG GCACAGGGTC AGTGTGGACC 3480
   AGGAACAACG AGGTCGTCGT ACTGACAGCG TCACACGTGG TTGGCCGCGC TAACATGGCC 3540
   ACTCTGAAGA TCGGTGACGC AATGCTGACT CTGACTTTCA AAAAGAATGG CGACTTCGCC 3600
   GAGGCAGTGA CGACACAGTC CGAGCTCCCA GGCAATTGGC CACAGTTGCA TTTCGCCCAA 3660
   CCAACAACCG GGCCCGCTTC ATGGTGCACT GCCACAGGAG ATGAAGAAGG CTTGCTCAGT 3720
   GGCGAGGTTT GTCTGGCGTG GACTACTAGT GGCGACTCTG GATCTGCAGT GGTTCAGGGT 3780
   GACGCTGTGG TAGGGGTCCA CACCGGTTCG AACACAAGTG GTGTTGCCTA CGTGACCACC 3840
   CCAAGCGGAA AACTCCTTGG CGCCGACACC GTGACTTTGT CATCACTGTC AAAGCATTTC 3900
   ACAGGCCCTT TGACATCAAT CCCGAAGGAC ATCCCTGACA ACATTATTGC CGATGTTGAT 3960
   GCTGTTCCTC GTTCTCTGGC CATGCTGATT GATGGCTTAT CCAATAGAGA GAGCAGCCTT 4020
   TCTGGACCTC AGTTGTTGTT AATTGCTTGT TTTATGTGGT CTTATCTTAA CCAACCTGCT 4080
   TACTTGCCTT ATGTGCTGGG CTTCTTTGCC GCTAACTTCT TCCTGCCAAA AAGTGTTGGC 4140
   CGCCCTGTGG TCACTGGGCT TCTATGGTTG TGCTGCCTCT TCACACCGCT TTCCATGGGC 4200
   TTGTGCTTGT TCCATCTGGT CTGTGCTACC GTCACGGGAA ACGTGATATC TTTGTGGTTC 4260
   TACATCACTG CCGCTGGCAC GTCTTACCTT TCTGAGATGT GGTTCGGAGG CTATCCCACC 4320
   ATGTTGTTTG TGCCACGGTT CCTAGTGTAC CAGTTCCCCG GCTGGGCTAT TGGCACAGTA 4380
   CTAGCGGTAT GCAGCATCAC CATGCTGGCT GCTGCCCTCG GTCACACCCT GTTACTGGAT 4440
   GTGTTCTCCG CCTCAGGTCG CTTTGACAGG ACTTTCATGA TGAAATACTT CCTGGAGGGA 4500
   GGAGTGAAAG AGAGTGTCAC CGCCTCAGTC ACCCGCGCTT ATGGCAAACC AATTACCCAG 4560
   GAGAGTCTCA CTGCAACATT AGCTGCCCTC ACTGATGATG ACTTCCAATT CCTCTCTGAT 4620
   GTGCTTGACT GTCGGGCCGT CCGATCGGCA ATGAATCTCG GTGCCGCTCT CACAAGTTTT 4680
   CAAGTGGCGC AGTATCGTAA CATCCTTAAT GCATCCTTGC AAGTCGATCG TGACGCTGCT 4740
   CGTAGTCGCA GACTAATGGC AAAACTGGCT GATTTTGCGG TTGAACAAGA AGTAACAGCT 4800
   GGAGACCGTG TTGTGGTTAT CGACGGTCTG GACCGCATGG CTCACTTCAA AGACGATTTG 4860
   GTGCTGGTTC CTTTGACCAC CAAAGTAGTA GGCGGTTCTA GGTGCACCAT TTGTGACGTC 4920
   GTTAAGGAAG AAGCCAATGA CACCCCAGTT AAGCCAATGC CCAGCAGGAG ACGCCGCAAG 4980
   GGCCTGCCTA AAGGTGCTCA GTTGGAGTGG GACCGTCACC AGGAAGAGAA GAGGAACGCC 5040
   GGTGATGATG ATTTTGCGGT CTCGAATGAT TATGTCAAGA GAGTGCCAAA GTACTGGGAT 5100
   CCCAGCGACA CCCGAGGCAC GACAGTGAAA ATCGCCGGCA CTACCTATCA GAAAGTGGTT 5160
   GACTATTCAG GCAATGTGCA TTACGTGGAG CATCAGGAAG ATCTGCTAGA CTACGTGCTG 5220
   GGCAAGGGGA GCTATGAAGG CCTAGATCAG GACAAAGTGT TGGACCTCAC AAACATGCTT 5280
   AAAGTGGACC CCACGGAGCT CTCCTCCAAA GACAAAGCCA AGGCGCGTCA CGTTGCTCAT 5340
   CTGCTGTTGG ATCTGGCTAA CCCAGTTGAG GCAGTGAATC AGTTAAACTG AGAGCGCCCC 5400
   ACATCTTTCC CGGCGATGTG GGGCGTCGGA CCTTTGCTGA CTCTAAAGAC AAGGGTTTCG 5460
   TGGCTCTACA CAGTCGCACA ATGTTTTTAG CTGCCCGGGA CTTTTTATTT AACATCAAAT 5520
   TTGTGTGCGA CGAAGAGTTC ACAAAGACCC CAAAAGACAC ACTGCTTGGG TACGTACGCG 5580
   CCTGCCCTGG TTACTGGTTT ATTTTCCGTC GTACGCACCG GTCGCTGATT GATGCATACT 5640
   GGGACAGTAT GGAGTGCGTT TACGCGCTTC CCACCATATC TGATTTTGAT GTGAGCCCAG 5700
   GTGACGTCGC AGTGACGGGC GAGCGATGGG ATTTTGAATC TCCCGGAGGA GGCCGTGCAA 5760
   AACGTCTCAC AGCTGATCTG GTGCACGCTT TTCAAGGGTT CCACGGAGCC TCTTATTCCT 5820
   ATGATGACAA GGTGGCAGCT GCTGTCAGTG GTGACCCGTA TCGGTCGGAC GGCGTCTTGT 5880
   ATAACACCCG TTGGGGCAAC ATTCCATATT CTGTCCCAAC CAATGCTTTG GAAGCCACAG 5940
   CTTGCTACCG TGCTGGATGT GAGGCCGTTA CCGACGGGAC CAACGTCATC GCAACAATTG 6000
   GGCCCTTCCC GGAGCAACAA CCCATACCGG ACATCCCAAA GAGCGTGCTT GACAACTGCG 6060
   CTGACATCAG CTGTGACGCT TTCATAGCGC CCGCTGCAGA GACAGCCCTG TGTGGAGATT 6120
   TAGAGAAATA CAACCTATCC ACGCAGGGTT TTGTGTTGCC TAGTGTTTTC TCCATGGTGC 6180
   GGGCGTACTT AAAAGAGGAG ATTGGAGACG CTCCACCACT CTACTTGCCA TCTACTGTAC 6240
   CATCTAAAAA TTCACAAGCC GGAATTAACG GCGCTGAGTT TCCTACAAAG TCTTTACAGA 6300
   GCTACTGTTT GATTGATGAC ATGGTGTCAC AGTCCATGAA AAGCAATCTA CAAACCGCCA 6360
   CCATGGCGAC TTGTAAACGG CAATACTGTT CCAAATACAA GATTAGGAGC ATTCTGGGCA 6420
   CCAACAATTA CATTGGCCTA GGTTTGCGTG CCTGCCTTTC GGGGGTTACG GCCGCATTCC 6480
   AAAAAGCTGG AAAGGATGGG TCACCGATTT ATTTGGGCAA GTCAAAATTC GACCCGATAC 6540
   CAGCTCCTGA CAAGTACTGC CTTGAAACAG ACCTGGAGAG TTGTGATCGC TCCACCCCGG 6600
   CTTTGGTGCG TTGGTTCGCT ACTAATCTTA TTTTTGAGCT AGCTGGCCAG CCCGAGTTGG 6660
   TGCACAGCTA CGTGTTGAAT TGCTGTCACG ATCTAGTTGT GGCGGGTAGT GTAGCATTCA 6720
   CCAAACGCGG GGGTTTGTCA TCTGGAGACC CTATCACTTC CATTTCCAAT ACCATCTATT 6780
   CATTGGTGCT GTACACCCAG CACATGTTGC TATGTGGACT TGAAGGCTAT TTCCCAGAGA 6840
   TTGCAGAAAA ATATCTTGAT GGCAGCCTGG AGCTGCGGGA CATGTTCAAG TACGTTCGAG 6900
   TGTACATCTA CTCGGACGAT GTGGTTCTAA CCACACCCAA CCAGCATTAC GCGGCCAGCT 6960
   TTGACCGCTG GGTCCCCCAC CTGCAGGCGC TGCTAGGTTT CAAGGTTGAC CCAAAGAAAA 7020
   CTGTGAACAC CAGCTCCCCT TCCTTTTTGG GCTGCCGGTT CAAGCAAGTG GACGGCAAGT 7080
   GTTATCTAGC CAGTCTTCAG GACCGCGTTA CACGCTCTCT GTTATACCAC ATTGGTGCAA 7140
   AGAATCCCTC AGAGTACTAT GAAGCTGCTG TTTCCATCTT TAAGGACTCC ATTATCTGCT 7200
   GTGATGAAGA CTGGTGGACG GACCTCCATC GACGTATCAG TGGCGCTGCG CGTACCGACG 7260
   GAGTTGAGTT CCCCACCATT GAAATGTTAA CATCCTTCCG CACCAAGCAG TATGAGAGTG 7320
   CCGTGTGCAC AGTTTGTGGG GCCGCCCCCG TGGCCAAGTC TGCTTGTGGA GGGTGGTTCT 7380
   GTGGCAATTG TGTCCCGTAC CACGCGGGTC ATTGTCACAC AACCTCGCTC TTCGCCAACT 7440
   GCGGGCACGA CATCATGTAC CGCTCCACTT ACTGCACAAT GTGTGAGGGT TCCCCAAAAC 7500
   AGATGGTACC AAAAGTGCCT CACCCGATCC TGGATCATTT GCTGTGCCAC ATTGATTACG 7560
   GCAGTAAAGA GGAACTAACT CTGGTAGTGG CGGATGGTCG AACAACATCA CCGCCCGGGC 7620
   GCTACAAAGT GGGTCACAAG GTAGTCGCCG TGGTTGCAGA TGTGGGAGGC AACATTGTGT 7680
   TTGGGTGCGG TCCTGGATCA CACATCGCAG TACCACTTCA GGATACGCTC AAGGGCGTGG 7740
   TGGTGAATAA AGCTCTGAAG AACGCCGCCG CCTCTGAGTA CGTGGAAGGA CCCCCTGGGA 7800
   GTGGGAAGAC TTTTCACCTG GTCAAAGATG TGCTAGCCGT GGTCGGTAGC GCGACCTTGG 7860
   TTGTGCCCAC CCACGCGTCC ATGCTGGACT GCATCAACAA GCTCAAACAA GCGGGCGCCG 7920
   ATCCATACTT TGTGGTGCCC AAGTATACAG TTCTTGACTT TCCCCGGCCT GGCAGTGGAA 7980
   ACATCACAGT GCGACTGCCA CAGGTCGGAA CCAGTGAGGG AGAAACCTTT GTGGATGAGG 8040
   TGGCCTACTT CTCACCAGTG GATCTGGCGC GCATTTTAAC CCAGGGTCGA GTCAAGGGTT 8100
   ACGGTGATTT AAATCAGCTC GGGTGCGTCG GACCCGCGAG CGTGCCACGT AACCTTTGGC 8160
   TCCGACATTT TGTCAGCCTG GAGCCCTTGC GAGTGTGCCA TCGATTCGGC GCTGCTGTGT 8220
   GTGATTTGAT CAAGGGCATT TATCCTTATT ATGAGCCAGC TCCACATACC ACTAAAGTGG 8280
   TGTTTGTGCC AAATCCAGAC TTTGAGAAAG GTGTAGTCAT CACCGCCTAC CACAAAGATC 8340
   GCGGTCTTGG TCACCGCACA ATTGATTCAA TTCAAGGCTG TACATTCCCT GTTGTGACTC 8400
   TTCGACTGCC CACACCCCAA TCACTGACGC GCCCGCGCGC AGTTGTGGCG GTTACTAGGG 8460
   CGTCTCAGGA ATTATACATC TACGACCCCT TTGATCAGCT TAGCGGGTTG TTGAAGTTCA 8520
   CCAAGGAAGC AGAGGCGCAG GACTTGATCC ATGGCCCACC TACAGCATGC CACCTGGGCC 8580
   AAGAAATTGA CCTTTGGTCC AATGAGGGCC TCGAATATTA CAAGGAAGTC AACCTGCTGT 8640
   ACACACACGT CCCCATCAAG GATGGTGTAA TACACAGTTA CCCTAATTGT GGCCCTGCCT 8700
   GTGGCTGGGA AAAGCAATCC AACAAAATTT CGTGCCTCCC GAGAGTGGCA CAAAATTTGG 8760
   GCTACCACTA TTCCCCAGAC TTACCAGGAT TTTGCCCCAT ACCAAAAGAA CTCGCTGAGC 8820
   ATTGGCCCGT AGTGTCCAAT GATAGATACC CGAATTGCTT GCAAATTACC TTACAGCAAG 8880
   TATGTGAACT CAGTAAACCG TGCTCAGCGG GCTATATGGT TGGACAATCT GTTTTCGTGC 8940
   AGACGCCTGG TGTGACATCT TACTGGCTTA CTGAATGGGT CGACGGCAAA GCGCGTGCTC 9000
   TACCAGATTC CTTATTCTCG TCCGGTAGGT TCGAGACTAA CAGCCGCGCT TTCCTCGATG 9060
   AAGCCGAGGA AAAGTTTGCC GCCGCTCACC CTCATGCCTG TTTGGGAGAA ATTAATAAGT 9120
   CCACCGTGGG AGGATCCCAC TTCATCTTTT CCCAATATTT ACCACCATTG CTACCCGCAG 9180
   ACGCTGTTGC CCTGGTAGGT GCTTCATTGG CTGGGAAAGC TGCTAAAGCT GCTTGCAGCG 9240
   TTGTTGATGT CTATGCTCCA TCATTTGAAC CTTATCTACA CCCTGAGACA CTGAGTCGCG 9300
   TGTACAAGAT TATGATCGAT TTCAAGCCGT GTAGGCTTAT GGTGTGGAGA AACGCGACCT 9360
   TTTATGTCCA AGAGGGTGTT GATGCAGTTA CATCAGCACT AGCAGCTGTG TCCAAACTCA 9420
   TCAAAGTGCC GGCCAATGAG CCTGTTTCAT TCCATGTGGC ATCAGGGTAC AGAACCAACG 9480
   CGCTGGTAGC GCCCCAGGCT AAAATTTCAA TTGGAGCCTA CGCCGCCGAG TGGGCACTGT 9540
   CAACTGAACC GCCACCTGCT GGTTATGCGA TCGTGCGGCG ATATATTGTA AAGAGGCTCC 9600
   TCAGCTCAAC AGAAGTGTTC TTGTGCCGCA GGGGTGTTGT GTCTTCCACC TCAGTGCAGA 9660
   CCATTTGTGC ACTAGAGGGA TGTAAACCTC TGTTCAACTT CTTACAAATT GGTTCAGTCA 9720
   TTGGGCCCGT GTGATGGGCT TAGTGTGGTC ACTGATTTCA AATTCTATTC AGACTATTAT 9780
   TGCTGATTTT GCTATTTCTG TGATTGATGC AGCGCTTTTC TTTCTCATGC TACTTGCATT 9840
   GGCTGTTGTT ACTGTGTTTC TTTTCTGGCT CATTGTTGCC ATCGGCCGCA GCTTGGTGGC 9900
   GCGGTGTTCA CGAGGTGCGC GTTACAGACC TGTTTAAGGA TTTGCAGTGC GACAACCTGC 9960
   GCGCGAAAGA TGCCTTCCCG AGTCTGGGAT ATGCTCTGTC GATTGGCCAG TCGAGGCTAT 10020
   CGTATATGCT GCAGGATTGG TTGCTTGCTG CGCACCGCAA GGAAGTTATG CCTTCCAATA 10080
   TCATGCCTAT GCCCGGTCTT ACTCCTGATT GCTTTGACCA TCTGGAGTCT TCTAGCTATG 10140
   CTCCATTTAT CAATGCCTAT CGGCAGGCAA TTTTGAGTCA ATACCCACAA GAGCTCCAGC 10200
   TCGAAGCCAT CAACTGTAAA TTGCTTGCTG TGGTTGCACC GGCATTGTAT CATAATTACC 10260
   ATCTAGCCAA TTTGACCGGA CCGGCCACAT GGGTCGTGCC TACAGTGGGC CAGTTGCACT 10320
   ATTATGCTTC TTCCTCTATT TTTGCTTCAT CTGTGGAAGT GTTGGCAGCA ATAATACTAC 10380
   TATTTGCATG CATACCACTA GTGACACGAG TGTACATCTC TTTTACGCGG CTAATGTCAC 10440
   CTTCCCGTCG CACTTCCAGC GGCACTTTGC CGCGGCGCAA GATTTTGTAG TGCACACGGG 10500
   TTATGAATAT GCCGGGGTCA CTATGTTAGT GCACTTGTTT GCCAACTTGG TTCTGACATT 10560
   TCCGAGCTTA GTTAATTGTT CCCGCCCTGT GAATGTCTTT GCTAATGCTT CTTGCGTGCA 10620
   AGTGGTTTGT AGTCATACCA ACTCAACTAC TGGCTTGGGT CAACTTTCTT TTTCCTTTGT 10680
   AGATGAAGAT CTACGGCTGC ATATCAGGCC TACTCTTATT TGTTGGTTTG CCTTGTTGTT 10740
   GGTGCACTTT CTACCCATGC CACGCTGCAG AGGCTCGTAA TTTTACTTAC ATTAGTCATG 10800
   GATTGGGCCA CGTGCACGGT CATGAGGGGT GTAGGAATTT TATTAATGTC ACTCATTCTG 10860
   CATTTCTTTA TCTTAATCCC ACCACTCCCA CTGCGCCGGC TATAACTCAT TGTTTACTTC 10920
   TGGTTCTGGC AGCCAAAATG GAACACCCAA ACGCTACTAT CTGGCTGCAG CTGCAGCCGT 10980
   TTGGGTATCA TGTGGCTGGC GATGTCATTG TCAACTTGGA AGAGGACAAG AGGCATCCTT 11040
   ACTTTAAACT TTTGAGAGCG CCGGCTTTAC CGCTTGGTTT TGTGGCTATA GTTTATGTTC 11100
   TTTTACGACT GGTACGTTGG GCTCAACGAT GTTATCTATG ATTGTATTGC TATTCTTGCT 11160
   TTGGGGTGCG CCATCACATG CTTACTTCTC ATACTACACC GCTCAGCGCT TCACAGACTT 11220
   CACCTTGTGT ATGCTGACGG ATCGCGGCGT TATTGCCAAT TTGCTGCGAT ATGATGAGCA 11280
   CACTGCTTTG TACAATTGTT CCGCCAGTAA AACCTGTTGG TATTGCACAT TCCTGGACGA 11340
   ACAGATTATC ACGTTTGGAA CCGATTGTGA TGACACCTAC GCGGTCCCAG TTGCTGAGGT 11400
   CCTGGAACAG GCGCATGGAC CGTACAGTGC GCTGTTTGAT GACATGCCCC CTTTTATTTA 11460
   CTATGGCCGT GAATTCGGCA TAGTTGTGTT GGATGTGTTT ATGTTCTATC CCGTTTTAGT 11520
   TCTGTTTTTC TTATCAGTAC TACCCTATGC TACGCTTATT CTTGAAATGT GTGTATCTAT 11580
   TCTGTTTATA ATCTATGGCA TTTACAGCGG GGCCTACTTG GCCATGGGCA TATTTGCGGC 11640
   CACGCTTGCT ATACATTCAA TTGTGGTCCT CCGCCAATTA CTGTGGTTAT GCCTGGCTTG 11700
   GCGATACCGC TGTACGCTTC ACGCGTCCTT TATATCAGCT GAGGGGAAAG TGTACCCCGT 11760
   AGACCCCGGA CTCCCGGTTG CCGCCGTGGG CAATCGGTTG TTAGTCCCAG GTAGGCCCAC 11820
   TATCGATTAT GCAGTGGCCT ACGGCAGCAA AGTCAACCTT GTGAGGTTGG GGGCAGCTGA 11880
   GGTATGGGAG CCATAGATTC ATTTTGTGGT GACGGGATTT TAGGTGAGTA TCTAGATTAC 11940
   TTTATTCTGT CCGTCCCACT CTTGCTGTTG CTTACTAGGT ATGTAGCATC TGGGTTAGTG 12000
   TATGTTTTGA CTGCCTTGTT CTATTCCTTT GTATTAGCAG CTTATATTTG GTTTGTTATA 12060
   GTTGGAAGAG CCTTTTCTAC TGCTTATGCT TTTGTGCTTT TGGCTGCTTT TCTGTTATTA 12120
   GTAATGAGGA TGATTGTGGG TATGATGCCT CGTCTTCGGT CCATTTTCAA CCATCGCCAA 12180
   CTGGTGGTAG CTGATTTTGT GGACACACCT AGTGGACCTG TTCCCATCCC CCGCTCAACT 12240
   ACTCAGGTAG TGGTTCGCGG CAACGGGTAC ACCGCAGTTG GTAACAAGCT TGTCGATGGC 12300
   GTCAAGACGA TCACGTCCGC AGGCCGCCTC TTTTCGAAAC GGACGGCGGC GACAGCCTAC 12360
   AAGCTACAAT GACCTACTGC GCATGTTTGG TCAGATGCGG GTCCGCAAAC CGCCCGCGCA 12420
   ACCCACTCAG GCTATTATTG CAGAGCCTGG AGACCTTAGG CATGATTTAA ATCAACAGGA 12480
   GCGCGCCACC CTTTCGTCGA ACGTACAACG GTTCTTCATG ATTGGGCATG GTTCACTCAC 12540
   TGCAGATGCC GGAGGACTCA CGTACACCGT CAGTTGGGTT CCTACCAAAC AAATCCAGCG 12600
   CAAAGTTGCG CCTCCAGCAG GGCCGTAAGA CGTGGATATT CTCCTGTGTG GCGTCATGTT 12660
   GAAGTAGTTA TTAGCCACCC AGGAACC 12687
(2) INFORMATION FOR SEQ ID NO: 2:
   (i) SEQUENCE CHARACTERISTICS:
      (A) LENGTH: 110 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 2:
   Met Ala Ser Arg Arg Ser Arg Pro Gln Ala Ala Ser Phe Arg Asn Gly
   1               5                   10                  15
   Arg Arg Arg Gln Pro Thr Ser Tyr Asn Asp Leu Leu Arg Met Phe Gly
               20                  25                  30
   Gln Met Arg Val Arg Lys Pro Pro Ala Gln Pro Thr Gln Ala Ile Ile
           35                  40                  45
   Ala Glu Pro Gly Asp Leu Arg His Asp Leu Asn Gln Gln Glu Arg Ala
       50                  55                  60
   Thr Leu Ser Ser Asn Val Gln Arg Phe Phe Met Ile Gly His Gly Ser
   65                  70                  75                  80
   Leu Thr Ala Asp Ala Gly Gly Leu Thr Tyr Thr Val Ser Trp Val Pro
                   85                  90                  95
   Thr Lys Gln Ile Gln Arg Lys Val Ala Pro Pro Ala Gly Pro
               100                 105                 110
(2) INFORMATION FOR SEQ ID NO: 3:
   (i) SEQUENCE CHARACTERISTICS:
      (A) LENGTH: 69 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 3:
   Met Ala Ser Arg Arg Ser Arg Pro Gln Ala Ala Ser Phe Arg Asn Gly
   1               5                   10                  15
   Arg Arg Arg Gln Pro Thr Ser Tyr Asn Asp Leu Leu Arg Met Phe Gly
               20                  25                  30
   Gln Met Arg Val Arg Lys Pro Pro Ala Gln Pro Thr Gln Ala Ile Ile
           35                  40                  45
   Ala Glu Pro Gly Asp Leu Arg His Asp Leu Asn Gln Gln Glu Arg Ala
       50                  55                  60
   Thr Leu Ser Ser Asn
   65
(2) INFORMATION FOR SEQ ID NO: 4:
   (i) SEQUENCE CHARACTERISTICS:
      (A) LENGTH: 28 amino acids
      (B) TYPE: amino acid
      (C) *STRANDEDNESS*: single
      (D) TOPOLOGY: linear
   (ii) MOLECULE TYPE: peptide
   (xi) SEQUENCE DESCRIPTION: SEQ ID NO: 4:
   Met Ala Ser Arg Arg Ser Arg Pro Gln Ala Ala Ser Phe Arg Asn Gly
   1               5                   10                  15
   Arg Arg Arg Gln Pro Thr Ser Tyr Asn Asp Leu Leu
               20                  25